Europäisches Patentamt

European Patent Office

Office européen des brevets

(19)

(11) Veröffentlichungsnummer: **0 067 608**
**B1**

(12) **EUROPÄISCHE PATENTSCHRIFT**

(45) Veröffentlichungstag der Patentschrift:
07.03.84

(51) Int. Cl.³: **C 07 C 103/52, A 61 K 37/02**

(21) Anmeldenummer: **82302812.1**

(22) Anmeldetag: **01.06.82**

(54) **CRF und Analoge.**

(30) Priorität: **08.06.81 US 271624**

(43) Veröffentlichungstag der Anmeldung:
**22.12.82 Patentblatt 82/51**

(45) Bekanntmachung des Hinweises auf die Patenterteilung:
**07.03.84 Patentblatt 84/10**

(84) Benannte Vertragsstaaten:
**AT BE CH DE FR GB IT LI LU NL SE**

(56) Entgegenhaltungen:
**Chemical Abstracts vol. 96, no. 15 12 April 1982 Columbus, Ohio, USA C. RIVIER et al. "In vivo corticotropin-releasing factor-induced secretion of adrenocorticotropin, beta-endorphin, and corticosterone" page 103, column 1, abstract no. 116224n**

(73) Patentinhaber: **THE SALK INSTITUTE FOR BIOLOGICAL STUDIES, 10010 North Torrey Pines Road, La Jolla California 92038 (US)**

(72) Erfinder: **Vale, Wylie Walker, Jr., 1643 Valdez, La Jolla, California (US)**
Erfinder: **Spiess, Joachim, 271 Cerro Street, Encinatas California (US)**
Erfinder: **Rivier, Catherine Laure, 9674 Blackgold Rd, La Jolla California (US)**
Erfinder: **Rivier,Jean Edouard Frederick, 9674 Blackgold Road, La Jolla California (US)**

(74) Vertreter: **Woodcraft, David Charles et al, BROOKES & MARTIN High Holborn House 52/54 High Holborn, London, WC1V 6SE (GB)**

CRF and Analogs

This invention is directed to peptides related to the hentetracontapeptide CRF and to the methods for pharmaceutical treatment of mammals using such peptides. More specifically, the invention relates to CRF and analogs of CRF, to pharmaceutical compositions containing CRF or such analogs and to methods of treatment of mammals using CRF or such analogs.

Experimental and clinical observations have supported the concept that the hypothalamus plays a key role in the regulation of adenohypophysial corticotropic cells secretory functions. Over 25 years ago, Guillemin, Rosenberg and Saffran and Schally independently demonstrated the presence of factors in hypothalamus which would increase the rate of ACTH secretion by the pituitary gland incubated in vitro or maintained in an organ culture. None of the secretagogs characterized to date meets the criteria expected of a physiologic corticotropin releasing factor, CRF. Accordingly the purification of a large CRF has been pursued. Starting material for the purification was an early side fraction of the 490,000 ovine hypothalamic fragments originally extracted in the Laboratory for Neuroendocrinology at The Salk Institute, as described in Burgus et al. Hypothalamus and Endocrine Functions (F. Labrie et al. eds.) Plenum, New York, 1976, p. 355. None of the earlier attempts at purification were felt to have obtained this large molecular weight CRF in a purity of greater than about 1%.

Sauvagine es a 40-residue, amidated generally similar peptide which was isolated from the skin of the South American frog Phyllomedusa sauvagei, was characterized by Erspamer et al. and was described in Regulatory Peptides, Vol. 2 (1981), pp. 1–13. Sauvagine has the formula:

pGlu-Gly-Pro-Pro-Ile-Ser-Ile-Asp-Leu-Ser-Leu-
Glu-Leu-Leu-Arg-Lys-Met-Ile-Glu-Ile-Glu-Lys-
Gln-Glu-Lys-Glu-Lys-Gln-Gln-Ala-Ala-Asn-Asn-
Arg-Leu-Leu-Leu-Asp-Thr-Ile-NH$_2$.

Sauvagine has been reported to have biological activity in lowering blood pressure in mammals and in stimulating the secretion of ACTH and β-endorphin.

CRF has now been isolated, purified and characterized as a hentetracontapeptide having the formula:

H-Ser-Gln-Glu-Pro-Pro-Ile-Ser-Leu-Asp-Leu-
Thr-Phe-His-Leu-Leu-Arg-Glu-Val-Leu-Glu-Met-
Thr-Lys-Ala-Asp-Gln-Leu-Ala-Gln-Gln-Ala-His-
Ser-Asn-Arg-Lys-Leu-Leu-Asp-Ile-Ala-NH$_2$.
It may alternatively be referred to as Amunine. The synthesis of the 41-residue peptide has been completed, and both the isolated CRF and the synthetic CRF have been found to stimulate ACTH and β-endorphin activies in vitro and in vivo. Synthetic CRF has been found to substantially lower blood pressure for an extended time period. As a result CRF has been made available in substantially pure form (i.e. substantially free of the remainder of a crude biological extract or of related synthetic replicates), and a purity of at least about 93% or higher is practically obtainable and useful for clinical testing.

Analogs of the 41-residue peptide CRF having the following formula have at least substantially the same biological activity:

Z-R$_1$-Pro-Pro-Ile-Ser-R$_8$-Asp-Leu-R$_{11}$-R$_{12}$-R$_{13}$-
Leu-Leu-Arg-R$_{17}$-R$_{18}$-R$_{19}$-Glu-R$_{21}$-R$_{22}$-Lys-R$_{24}$-R$_{25}$-
-R$_{26}$-R$_{27}$-R$_{28}$-Gln-Gln-Ala-R$_{32}$-R$_{33}$-Asn-Arg-R$_{36}$-
Leu-Leu-Asp-R$_{40}$-R$_{41}$-NH$_2$
wherein Z is hydrogen or an acyl group having 7 or less carbon atoms and/or a peptide up to 10 residues long; R$_1$ is Ser-Gln-Glu or pGlu-Gly or Gln-Glu or Glu or D-Ser-Gln-Glu or D-pGlu-Gly or desR$_1$; R$_8$, R$_{12}$, R$_{19}$, R$_{24}$ and R$_{40}$ are selected from the group consisting of Leu, Ile, Ala, Gly, Val, Nle, Phe and Gln; R$_{11}$ is Thr or Ser; R$_{13}$ is His, Tyr or Glu; R$_{17}$ is Glu or Lys; R$_{18}$ is Val or Met; R$_{21}$ is Met, Met(O), Ile, Ala, Leu, Gly, Nle, Val, Phe or Gln; R$_{22}$ is Thr or Glu; R$_{25}$ is Asp or Glu; R$_{26}$ is Gln or Lys; R$_{27}$ is Leu, Ile, Ala, Gly, Val, Nle, Phe, Asp, Asn, Gln or Glu; R$_{28}$ is Ala or Lys; R$_{32}$ is His, Tyr or Ala; R$_{33}$ is Ser, Asn, Thr or Ala; R$_{36}$ is Lys or Leu; R$_{41}$ is Ala, Ile, Gly, Val, Leu, Nle, Phe, Gln or des R$_{41}$, provided however that when R$_{13}$ is His, then R$_{17}$ is Glu, R$_{18}$ is Val, R$_{22}$ is Thr, R$_{26}$ is Gln, R$_{28}$ is Ala, and R$_{36}$ is Lys; and provided that when R$_{13}$ is Glu, R$_{17}$ is Lys, R$_{18}$ is Met, R$_{22}$ is Glu, R$_{26}$ is Lys, R$_{28}$ is Lys, R$_{32}$ is Ala and R$_{36}$ is Leu, then either R$_1$ is not pGlu-Gly or R$_8$ is not Ile or R$_{11}$ is Thr or R$_{12}$ is not Leu or R$_{19}$ is not Ile or R$_{21}$ is not Ile or R$_{24}$ is not Gln or R$_{27}$ is not Glu or R$_{33}$ is not Asn or R$_{40}$ is not Thr or R$_{41}$ is not Ile.

Pharmaceutical compositions in accordance with the invention include CRF or its analogs, or nontoxic addition salts thereof, dispersed in a pharmaceutically acceptable liquid or solid carrier. The administration of such peptides or pharmaceutically acceptable addition salts thereof to mammals in accordance with the invention may be carried out for the regulation of secretion of ACTH, β-endorphin, β-lipotropin, and corticosterone and/or for the lowering of blood pressure and/or for affecting mood, behavioral and gastrointestinal functions.

CRF has been isolated from ovine hypothalamic extracts purified and characterized. The nomenclature used to define the peptides is that specified by Schroder & Lubke, 'The Peptides', Academic Press (1965) wherein, in accordance with conventional representation, the amino group appears to the left and the carboxyl group to the right. Where the amino acid residue has isomeric forms, it is the L-form of the amino acid that is represented unless otherwise expressly indicated.

The invention provides CRF and analogs of CRF having the following formula:

Z-R$_1$-Pro-Pro-Ile-Ser-R$_8$-Asp-Leu-R$_{11}$-R$_{12}$-R$_{13}$-Leu-Leu-Arg-R$_{17}$-R$_{18}$-R$_{19}$-Glu-R$_{21}$-R$_{22}$-Lys-R$_{24}$-R$_{25}$-R$_{26}$-R$_{27}$-R$_{28}$-Gln-Gln-Ala-R$_{32}$-R$_{33}$-Asn-Arg-R$_{36}$-Leu-Leu-Asp-R$_{40}$-R$_{41}$-NH$_2$

wherein Z is hydrogen or an acyl group having 7 or less carbon atoms and/or a peptide up to 10 residues long; R$_1$ is Ser-Gln-Glu or pGlu-Gly or Gln-Glu or Glu or D-Ser-Gln-Glu or D-pGlu-Gly or desR$_1$; R$_8$, R$_{12}$, R$_{19}$, R$_{24}$ and R$_{40}$ are selected from the group consisting of Leu, Ile, Ala, Gly, Val, Nle, Phe and Gln; R$_{11}$ is Thr or Ser; R$_{13}$ is His, Tyr or glu; R$_{17}$ is Glu or Lys; R$_{18}$ is Val or Met; R$_{21}$ is Met, Met(O), Ile, Ala, Leu, Gly, Nle, Val, Phe or Gln; R$_{22}$ is Thr or Glu; R$_{25}$ is Asp or Glu; R$_{26}$ is Gln or Lys; R$_{27}$ is Leu, Ile, Ala, Gly, Val, Nle, Phe, Asp, Asn, Gln or Glu; R$_{28}$ is Ala or Lys; R$_{32}$ is His, Tyr or Ala; R$_{33}$ is Ser, Asn, Thr or Ala; R$_{36}$ is Lys or Leu; R$_{41}$ is Ala, Ile, Gly, Val, Leu, Nle, Phe, Gln or des R$_{41}$, provided however that when R$_{13}$ is His, then R$_{17}$ is Glu, R$_{18}$ is Val, R$_{22}$ is Thr, R$_{26}$ is Gln, R$_{28}$ is Ala, and R$_{36}$ is Lys; and provided that when R$_{13}$ is Glu, R$_{17}$ is Lys, R$_{18}$ is Met, R$_{22}$ is Glu, R$_{26}$ is Lys, R$_{28}$ is Lys, R$_{32}$ is Ala and R$_{36}$ is Leu, then either R$_1$ is not pGlu-Gly or R$_8$ is not Ile or R$_{11}$ is Thr or R$_{12}$ is not Leu or R$_{19}$ is not Ile or R$_{21}$ is not Ile or R$_{24}$ is not Gln or R$_{27}$ is not Glu or R$_{33}$ is not Asn or R$_{40}$ is not Thr or R$_{41}$ is not Ile.

The peptides are synthesized by a suitable method, such as by exclusively solid-phase techniques, by partial solid-phase techniques, by fragment condensation or by classical solution addition. CRF and certain analogs may also be synthesized by recently developed recombinant DNA techniques.

Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side-chain protecting groups.

Also considered to be within the scope of the present invention are intermediates of the formula:

X$^1$-R$_1$-Pro-Pro-Ile-Ser(X$^2$)-R$_8$-Asp(X$^5$)-Leu-R$_{11}$ (X$^2$)-R$_{12}$-R$_{13}$-Leu-Leu-Arg(X$^3$)-R$_{17}$-R$_{18}$-R$_{19}$-Glu(X$^5$)-R$_{21}$-R$_{22}$-Lys(X$^6$)-R$_{24}$-R$_{25}$-R$_{26}$-R$_{27}$-R$_{28}$-Gln(X$^4$)-Gln (X$^4$)-Ala-R$_{32}$-R$_{33}$-Asn(X$^4$)-Arg(X$^3$)-R$_{36}$-Leu-Leu-Asp(X$^5$)-R$_{40}$-R$_{41}$-X$^7$

wherein: the R-groups are as hereinbefore defined; X$^1$ is either hydrogen or an α-amino protecting group. The α-amino protecting groups contemplated by X$^1$ are those known to be useful in the art in the step-wise synthesis of polypeptides. Among the classes of α-amino protecting groups covered by X$^1$ are (1) acyl-type protecting groups, such as formyl, acrylyl(Acr), benzoyl(Bz) and acetyl(Ac) which are preferably used only at the N-terminal; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl; (4) cycloalkyl urethan-type protecting groups, such as cyclopentyloxycarbonyl, adamantyloxycarbonyl, and cyclohexyloxycarbonyl; and (5) thiourethan-type protecting groups, such as phenylthiocarbonyl. The preferred α-amino protecting group is BOC.

X$^2$ is a protecting group for the hydroxyl group of Thr and Ser and is preferably selected from the class consisting of acetyl(Ac), benzoyl(Bz), tert-butyl, triphenylmethyl(trityl), tetrahydropyranyl, benzyl ether(Bzl) and 2,6-dichlorobenzyl (DCB). The most preferred protecting group is Bzl. X$^2$ can be hydrogen, which means there is no protecting group on the hydroxyl group.

X$^3$ is a protecting group for the guanidino group of Arg preferably selected from the class consisting of nitro, p-toluenesulfonyl(Tos), Z, adamantyloxycarbonyl and BOC, or is hydrogen. Tos is most preferred.

X$^4$ is hydrogen or a protecting group for the amido group of Asn or Gln and is preferably xanthyl(Xan).

X$^5$ is hydrogen or an ester-forming protecting group for the β- or α-carboxyl group of Asp or Glu, preferably selected from the class consisting of benzyl, 2,6-dichlorobenzyl, methyl, ethyl and t-butyl ester. OBzl is most preferred.

X$^6$ is hydrogen or a protecting group for the side chain amino substituent of Lys. Illustrative of suitable side chain amino protecting groups are Z, 2-chlorobenzyloxycarbonyl(2-Cl-Z), Tos, t-amyloxycarbonyl(Aoc), BOC and aromatic or aliphatic urethan-type protecting groups as specified hereinbefore.

When Met is present, the sulfur may be protected, if desired, with oxygen. When His is present, the imidazole nitrogen can be protected with Tos or 2,4-dinitrophenyl(DNP). When Tyr is present, the hydroxyl group may be protected with DCB.

The selection of a side chain amino protecting group is not critical except that it must be one which is not removed during deprotection of the α-amino groups during the synthesis. Hence, the α-amino protecting groups and the side chain amino protecting group cannot be the same.

X$^7$ is selected from the class consisting of OH, OCH$_3$, amides, hydrazides, esters and an amide anchoring bond used in solid phase synthesis for linking to a solid resin support, represented by the formulae:

-O-CH$_2$-benzyl-polyamide resin support, -NH-benzhydrylamine (BHA) resin support, and -NH-paramethylbenzhydrylamine (MBHA) resin support.

The polyamide polymer is commerically available and is discussed in detail in Bioorganic Chemistry, 8, 351–370 (1979) where a preferred version of it is discussed in Figure 6. Use of BHA or MBHA resin is preferred, and cleavage gives the CRF amide or CRF analog amide.

In the formula for the intermediate, at least one of $X^1$, $X^2$, $X^3$, $X^4$, $X^5$ and $X^6$ is a protecting group. Depending upon the particular amino acids chosen for the R-groups, they may also have a protecting group attached as specified hereinbefore and as generally known in the art and mentioned hereinbefore. In selecting a particular side chain protecting group to be used in the synthesis of the peptides, the following rules are followed: (a) the protecting group must be stable to the reagent and under the reaction conditions selected for removing the α-amino protecting group at each step of the synthesis, (b) the protecting group must retain its protecting properties and not be split off under coupling conditions and (c) the side chain protecting group must be removable, upon the completion of the synthesis containing the desired amino acid sequence, under reaction conditions that will not alter the peptide chain.

For the acyl group represented by Z, acetyl, formyl, acrylyl and benzoyl are preferred. For the 1 to 10 amino acid peptide which may be optionally included without adversely affecting the potency, any amino acids may be used, but the L- or D- forms of the naturally occurring amino acids would normally be used.

The peptides are preferably prepared using solid phase synthesis, such as that described by Merrifield, J. Am. Chem. Soc., 85, p. 2149 (1964), although other equivalent chemical syntheses known in the art can also be used as previously mentioned. Solid-phase synthesis is commenced from the C-terminal end of the peptide by coupling a protected α-amino acid to a suitable resin as generally set forth in U.S. Patent No. 4,244,946 issued Jan. 21, 1981 to Rivier et al., the disclosure of which is incorporated herein by reference. Such a starting material for CRF can be prepared by attaching α-amino-protected Ala to a BHA resin.

Ala protected by BOC coupled to the BHA resin using methylene chloride and dimethylformamide (DMF). Following the coupling of BOC-Ala to the resin support, the α-amino protecting group is removed, as by using trifluoroacetic acid(TFA) in methylene chloride, TFA alone or with HCl in dioxane. Preferably 50 weight % TFA in methylene chloride is used with 0–5 weight % 1,2-ethanedithiol. The deprotection is carried out at a temperature between about 0°C and room temperature. Other standard cleaving reagents and conditions for removal of specific α-amino protecting groups may be used as described in Schroder & Lubke, 'The Peptides', 1 pp. 72–75 (Academic Press 1965).

After removal of the α-amino protecting group of Ala, the remaining α-amino- and side chain-protected amino acids are coupled step-wise in the desired order to obtain the intermediate compound defined hereinbefore. As an alternative to adding each aminoacid separately in the synthesis, some of them may be coupled to one another prior to addition to the solid phase reactor. The selection of an appropriate coupling reagent is within the skill of the art. Particularly suitable as a coupling reagent is N,N'-dicyclohexyl carbodiimide (DCCI).

The activating reagents used in the solid phase synthesis of the peptides are well known in the peptide art. Examples of suitable activating reagents are carbodiimides, such as N,N'-diisopropyl carbodiimide and N-ethyl-N'-(3-dimethylaminopropyl)carbodiimide. Other activating reagents and their use in peptide coupling are described by Schroder & Lubke, supra, in Chapter I-II and by Kapoor, J. Phar. Sci., 59, pp. 1–27 (1970).

Each protected amino acid or amino acid sequence is introduced into the solid phase reactor in about a fourfold excess, and the coupling is carried out in a medium of dimethylformamide(DMF):CH$_2$Cl$_2$ (1:1) or in DMF or CH$_2$Cl$_2$ alone. In instances where the coupling is carried out manually, the success of the coupling reaction at each stage of the synthesis is monitored by the ninhydrin reaction, as described by E. Kaiser et al., Anal. Biochem. 34, 595 (1970). In cases where incomplete coupling occurs, the coupling procedure is repeated before removal of the α-amino protecting group prior to the coupling of the next amino acid. The coupling reactions can be performed automatically, as on a Beckman 990 automatic synthesizer, using a program such as that reported in Rivier et al., Biopolymers, 1978, 17, pp. 1927–1938.

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups $X^2$, $X^3$, $X^4$, $X^5$ and $X^6$ and the α-amino protecting group $X^1$ (unless it is an acyl group which is intended to be present in the final peptide), to obtain the peptide. When using hydrogen fluoride for cleaving, anisole and methylethyl sulfide are included in the reaction vessel as scavengers. When Met is present in the sequence, the BOC protecting group may be cleaved with trifluoroacetic acid(TFA)/ethanedithiol prior to cleaving the peptide from the resin to eliminate S-alkylation.

The following Example sets forth the preferred method for synthesizing analogs of CRF by the solid-phase technique.

Example 1

The synthesis of the CRF having the formula:
H-Ser-Gln-Glu-Pro-Pro-Ile-Ser-Leu-Asp-Leu-
Thr-Phe-His-Leu-Leu-Arg-Glu-Val-Leu-Glu-Met-
Thr-Lys-Ala-Asp-Gln-Leu-Ala-Gln-Gln-Ala-His-
Ser-Asn-Arg-Lys-Leu-Leu-Asp-Ile-Ala-NH$_2$
and a molecular weight of 4666 is conducted in a stepwise manner on a benzhydrylamine hydrochloride resin, such as available from Bachem, Inc., having a substitution range of about 0.1 to 0.5 mmoles/gm. resin. The synthesis is performed on an automatic Beckman 990A peptide synthesizer. Coupling of BOC-Ala results in the substitution of about 0.35 mmol. Ala per gram of resin. All solvents that are used are carefully degassed, preferably by sparging with an inert gas, e.g. helium or nitrogen, to insure the absence of oxygen that might undesirably oxidize the sulfur of the Met residue.

After deprotection and neutralization, the peptide chain is built step-by-step on the resin. Generally, one to two mmol. of BOC-protected amino acid in methylene chloride is used per gram of resin, plus one equivalent of 2 molar DCCI in methylene chloride, for two hours. When BOC-Arg(Tos) is being coupled, a mixture of 50% DMF and methylene chloride is used. Bzl is used as the hydroxyl side-chain protecting group for Ser and Thr. P-nitrophenyl ester(ONp) is used to activate the carboxyl end of Asn or Gln, and for example, BOC-Asn(ONp) is coupled overnight using one equivalent of HOBt in a 50% mixture of DMF and methylene chloride. The amido group of Asn or Gln is protected by Xan when DCC coupling is used instead of the active ester method. 2-Cl-Z is used as the protecting group for the Lys side chain. Tos is used to protect the guanidino group of Arg and the imidazole group of His, and the side chain carboxyl group of Glu or Asp is protected by OBzl. At the end of the synthesis, the following composition is obtained
BOC-Ser(Bzl)-Gln(Xan)-Glu(OBzl)-Pro-Pro-Ile-
Ser(Bzl)-Leu-Asp(OBzl)-Leu-Thr(Bzl)-Phe-His
(Tos)-Leu-Leu-Arg(Tos)-Glu(OBzl)-Val-Leu-Glu
(OBzl)-Met-Thr(Bzl)-Lys(2-Cl-Z)-Ala-Asp(OBzl)-
Gln(Xan)-Leu-Ala-Gln(Xan)-Gln(Xan)-Ala-His
(Tos)-Ser(Bzl)-Asn(Xan)-Arg(Tos)-Lys(2-Cl-Z)-
Leu-Leu-Asp(OBzl)-Ile-Ala-resin support. Xan may have been partially or totally removed by TFA treatment used to deblock the α-amino protecting group.

In order to cleave and deprotect the resulting protected peptide-resin, it is treated with 1.5 ml. anisole, 0.5 ml. of methyl ethyl sulfide and 15 ml. hydrogen fluoride (HF) per gram·of peptide-resin, first at −20°C. for 20 min. and then at 0°C. for one-half hour. After elimination of the HF under high vacuum, the resin-peptide is washed alternately with dry diethyl ether and chloroform, and the peptides are then extracted with de-gassed 2N aqueous acetic acid and separated from the resin by filtration.

The peptide is purified by gel permeation followed by semi-preparative HPLC as described in Rivier et al., Peptides: Structure and Biological Function (1979) pp. 125–128. The chromatographic fractions are carefully monitored by HPLC, and only the fractions showing substantial purity were pooled.

Specific optical rotation of the CRF peptide, which was synthesized and purified in the foregoing manner, was measured on a Perkin Elmer Model 141 as $[\alpha]_D^{22°} = -77.5° \pm 1.0$ (c=1 in 1% acetic acid) without correcting for the presence of H$_2$O and TFA) and had a purity of about 96%. To check whether the precise sequence was achieved, the CRF peptide was hydrolyzed in sealed evacuated tubes containing 4N methanesulfonic acid and 0.2% tryptamine for 24 hours at 110°C. Amino acid analyses of the hydrolysates using a Beckman 121 MB amino acid analyzer showed the following amino acid ratios: Asp(4.02), Thr(1.85), Ser(2.76), Glu(7.0), Pro(1.58), Ala(4.03), Val(0.96), Met(0.95), Ile(1.93), Leu(8.15), Phe(1.00), Lys(2.00), His(1.95) and Arg(1.98), which confirmed that the 41-residue peptide structure had been obtained.

Example 2

CRF was extracted, isolated and purified in the following manner. 490,000 ovine hypothalamic fragments were extracted in ethanol-acetic acid-chloroform, defatted with a mixture of ethyl ether and petroleum ether and subjected to repeated shake-out with the partition system 0.1% acetic acid: n-butanol: pyridine (11:5:3). the combined aqueous phases show ACTH-releasing activity.

Following dialysis (Spectrapor 3) against 2N HOAc, about 350,000 fragment equivalents of the retentates weighing 15 grams were subjected to gel filtration on Sephadex G-50. The bulk of material was chromatographed at cold room temperatures in 9 successive runs using a 3.1 × 150 cm. G-50 column eluted with 2N HOAc. The zone showing the most significant ACTH-releasing activity eluted at about 1.3 $V_e/V_o$.

A portion of the material from this zone, about 130,000 fragment equivalents, was subjected to ion exchange on SP-Sephadex. The sample was applied in 0.01 M ammonium formate buffer, pH 3.2 and eluted with a linear gradient of the application buffer to 1:5 M ammonium formate, pH 7.0. ACTH releasing activity was weakly absorbed. The ACTH releasing fraction from SP-Sephadex and the remainder of the zone from Sephadex-G-50 were dissolved in 6 M guanidine HCl/HOAc, pH 2.5, heated 5 min. at 90°, then chromatographed on Biogel P-10 with 4 M guanidine HCl/HOAc, ph 2.5. The ACTH-releasing fractions were pooled and purified further by successive high pressure liquid chromatographic (HPLC) steps which included: 1) Reverse phase HPLC on Bondapak CN (Waters and Associates) using a gradient of decreasing triethylammonium phosphate (TEAP) and increasing acetonitrile; 2) Reverse phase HPLC on Bondapak C$_{18}$ (Waters and Associates) with TEAP/acetonitrile; and either 3a) Reverse phase HPLC on Bondapak CN with gradients of triethylammonium formate and acetonitrile, or 3b) Reverse phase HPLC on $C_{18}$ with gradients of trifluoroacetic acid and acetonitrile. Lyophilized active zones from both HPLC steps 3a or 3b were used for composition and structural analysis which gave the following sequence:
H-Ser-Gln-Glu-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-NH$_2$.

Example 3

The synthetic and the natural CRF were examined for their effects on the secretion of ACTH and β-endorphin in vitro and the synthetic CRF was also examined in vivo. The high potency of synthetic and natural CRF to stimulate the secretion of ACTH and β-endorphin by cultured rat pituitary cells was measured. Minimal and half-maximal responses were observed at about 10 picomolar and about 100 picomolar of synthetic CRF, respectively. The secretory response to maximal (>5nM) concentrations of CRF is at a plateau level. In vivo doses from 30 ng to 3 μg/kg of body weight rapidly elevated ACTH and β-endorphin-like (β-END-LI) secretion 5–20 fold.

Synthetic CRF has been shown to be a powerful stimulator of ACTH and β-END-LI secretion in vivo in several rat preparations. Plasma levels of ACTH and β-END-LI are elevated for at least 5–20 minutes following the intravenous administration of CRF to nembutal-anesthesized male rats and to quiescent male or female rats with indweling intravenous cannulae. In addition, CRF is found to have a dramatic effect upon blood pressure in rats and dogs. Mean cartoid blood pressure in a urethane-anesthesized rat fell from 87 to 72±2 mm Hg and remained at that level for 30 minutes following an injection equal to 3 μg CRF/kg body weight; the subsequent administration of an injection equal to 30 μg CRF/kg body weight lowered mean blood pressure to 42±3 mm Hg for more than 2 hours.

Example 4

The peptide [des Ser¹-Gln²-Glu³]-CRF having the formula:

H-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure.

Example 5

The peptide [des pGlu¹-Gly²]-sauvagine having the formula:

H-Pro-Pro-Ile-Ser-Ile-Asp-Leu-Ser-Leu-Glu-Leu-Leu-Arg-Lys-Met-Ile-Glu-Ile-Glu-Lys-Gln-Glu-Lys-Glu-Lys-Gln-Gln-Ala-Ala-Asn-Asn-Arg-Leu-Leu-Leu-Asp-Thr-Ile-NH$_2$
is synthesized. Testing in accordance with the general procedure set forth in Example 3 shows that it likewise stimulates the secretion of ACTH and β-END-LI and causes a very significant lowering of blood pressure.

Example 6

The peptide [des Ala⁴¹]-CRF having the formula:

H-Ser-Gln-Glu-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-NH$_2$
is synthesized. Testing in accordance with the general procedure set forth in Example 3 shows that it likewise stimulates the secretion of ACTH and β-END-LI and causes a very significant lowering of blood pressure.

Example 7

The peptide [Acetyl-Ala¹]-CRF having the formula:

Ac-Ala-Gln-Glu-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure.

Example 8

The peptide [Ile¹⁹, Glu²⁵]-CRF having the formula:

H-Ser-Gln-Glu-Pro-Pro-Ile-Ser-Leu-Asp-Leu-Thr-Phe-His-Leu-Leu-Arg-Glu-Val-Ile-Glu-Met-Thr-Lys-Ala-Glu-Gln-Leu-Ala-Gln-Gln-Ala-His-Ser-Asn-Arg-Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure.

Example 9

The peptide [Tyr¹³, Nle²¹]-CRF having the formula:

H-Ser-Gln-Glu-Pro-Pro-Ile-Ser-Leu-Asp-Leu-Thr-Phe-Tyr-Leu-Leu-Arg-Glu-Val-Leu-Glu-Nle-Thr-Lys-Ala-Asp-Gln-Leu-Ala-Gln-Gln-Ala-His-Ser-Asn-Arg-Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure to a greater extent than CRF.

Example 10

The peptide [Ile[8], Ser[11], Asn[33]]-CRF having the formula:

H-Ser-Gln-Glu-Pro-Pro-Ile-Ser-Ile-Asp-Leu-Ser-
Phe-His-Leu-Leu-Arg-Glu-Val-Leu-Glu-Met-Thr-
Lys-Ala-Asp-Gln-Leu-Ala-Gln-Gln-Ala-His-Asn-
Asn-Arg-Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure.

Example 11

The peptide [Acrylyl-Phe-Gly-Ala[1], Ser[2]]-CRF having the formula:

Acr-Phe-Gly-Ala-Ser-Glu-Pro-Pro-Ile-Ser-Leu-
Asp-Leu-Thr-Phe-His-Leu-Leu-Arg-Glu-Val-Leu-
Glu-Met-Thr-Lys-Ala-Asp-Gln-Leu-Ala-Gln-Gln-
Ala-His-Ser-Asn-Arg-Lys-Leu-Leu-Asp-Ile-Ala-
NH$_2$
is synthesized. Testing in accordance with the general procedure set forth in Example 3 shows that it likewise stimulates the secretion of ACTH and β-END-LI and causes a very significant lowering of blood pressure.

Example 12

The peptide [Benzoyl-Ala[1], des Gln[2], Leu[12]]-CRF having the formula:

Bz-Ala-Glu-Pro-Pro-Ile-Ser-Leu-Asp-Leu-Thr-
Leu-His-Leu-Leu-Arg-Glu-Val-Leu-Glu-Met-Thr-
Lys-Ala-Asp-Gln-Leu-Ala-Gln-Gln-Ala-His-Ser-
Asn-Arg-Lys-Leu-Leu-Asp-Ile-Ala-NH$_2$
is synthesized. Testing in accordamce with the general procedure set forth in Example 3 shows that it likewise stimulates the secretion of ACTH and β-END-LI and causes a very significant lowering of blood pressure.

Example 13

The peptide [Ala[21], Thr[33], Nle[41]]-CRF having the formula:

H-Ser-Gln-Glu-Pro-Pro-Ile-Ser-Leu-Asp-Leu-
Thr-Phe-His-Leu-Leu-Arg-Glu-Val-Leu-Glu-Ala-
Thr-Lys-Ala-Asp-Gln-Leu-Ala-Gln-Gln-Ala-His-
Thr-Asn-Arg-Lys-Leu-Leu-Asp-Ile-Nle-NH$_2$
is synthesized. Testing in accordance with the general procedure set forth in Example 3 shows that it likewise stimulates the secretion of ACTH and β-END-LI and cause a very significant lowering of blood pressure.

Example 14

The peptide [Ala[39], Ala[40]-sauvagine having the formula:

Glu-Gly-Pro-Pro-Ile-Ser-Ile-Asp-Leu-Ser-Leu-
Glu-Leu-Leu-Arg-Lys-Met-Ile-Glu-Ile-Glu-Lys-
Gln-Glu-Lys-Glu-Lys-Gln-Gln-Ala-Ala-Asn-Asn-
Arg-Leu-Leu-Leu-Asp-Ala-Ala-NH$_2$
is synthesized. Testing in accordance with the general procedure set forth in Example 3 shows that it likewise stimulates the secretion of ACTH and β-END-LI and causes a very significant lowering of blood pressure.

Example 15

The peptide [Gly[26]]-sauvagine having the formula:

pGlu-Gly-Pro-Pro-Ile-Ser-Ile-Asp-Leu-Ser-Leu-
Glu-Leu-Leu-Arg-Lys-Met-Ile-Glu-Ile-Glu-Lys-
Gln-Glu-Lys-Gly-Lys-Gln-Gln-Ala-Ala-Asn-Asn-
Arg-Leu-Leu-Leu-Asp-Thr-Ile-NH$_2$
is synthesized. Testing in accordance with the general procedure set forth in Example 3 shows that it likewise stimulates the secretion of ACTH and β-END-LI and causes a very significant lowering of blood pressure.

Example 16

The peptide [Acetyl-Pro[4]]-CRF having the formula:

Ac-Pro-Pro-Ile-Ser-Leu-Asp-Leu-Thr-Phe-His-
Leu-Leu-Arg-Glu-Val-Leu-Glu-Met-Thr-Lys-Ala-
Asp-Gln-Leu-Ala-Gln-Gln-Ala-His-Ser-Asn-Arg-
Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure to a greter extent than CRF.

Example 17

The peptide [Nle[21], Tyr[32]]-CRF having the formula:

H-Ser-Gln-Glu-Pro-Pro-Ile-Ser-Leu-Asp-Leu-
Thr-Phe-His-Leu-Leu-Arg-Glu-Val-Leu-Glu-Nle-
Thr-Lys-Ala-Asp-Gln-Leu-Ala-Gln-Gln-Ala-Tyr-
Ser-Asn-Arg-Lys-Leu-Leu-Asp-Ile-Ala-NH$_2$
is synthesized. Testing in accordance with the general procedure set forth in Example 3 shows that it likewise stimulates the secretion of ACTH and β-END-LI and causes a very significant lowering of blood pressure to a greater extent than CRF.

Example 18

The peptide [Met(O)[21]]-CRF having the formula:
H-Ser-Gln-Glu-Pro-Pro-Ile-Ser-Leu-Asp-Leu-
Thr-Phe-His-Leu-Leu-Arg-Glu-Val-Leu-Glu-
Met(O)-Thr-Lys-Ala-Asp-Gln-Leu-Ala-Gln-Gln-
Ala-His-Ser-Asn-Arg-Lys-Leu-Leu-Asp-Ile-Ala-
NH$_2$
is synthesized. Testing in accordance with the general procedure set forth in Example 3 shows that it likewise stimulates the secretion of ACTH and β-END-LI and causes a very significant lowering of blood pressure.

It is of interest that CRF and its analogs exhibited such an extreme lowering of blood pressure. As a result, these peptides may be particularly valuable for the treatment of high blood pressure conditions and also for the treatment of patients who are to undergo certain types of surgery.

As CRF profoundly stimulates the pituitary-adrenalcortical axis, CRF or its analogs should be useful to stimulate the functions of this axis in some types of patients with low endogenous glucocorticoid production. For example, CRF should be useful in restoring pituitary-adrenal function in patients having received exogenous glucocorticoid therapy whose adrenalcortical functions remain suppressed.

Most other regulatory peptides have been found to have effects upon the central nervous system and upon the gastrointestinal tract. Because ACTH and β-END secretion is the «sine qua non» of an animal's response to stress, it is likely that CRF should have significant effects on the brain as a mediator or limiter of the body's stress response. Accordingly, CRF may also find application in modifying the mood and behavior of normal and mentally disordered individuals. Because CRF and its analogs elevate the levels of ACTH, β-END, β-lipotropin and corticosterone, its administration can be used to induce their effects on the brain and its periphery to thereby influence memory, mood, pain appreciation, etc., and more specifically, alertness, depression and/or anxiety.

It is also believed that relatively larger amounts of CRF or some of its more potent analogs may function in a manner similar to the way that GnRH superagonists inhibit reproductive functions. Thus, these peptides may be able to desensitize CRF target organs and thus be valuable in the treatment of subjects with Cushing's disease and similar disorders.

CRF, its analogs or the nontoxic addition salts thereof, combined with a pharmaceutically acceptable carrier to form a pharmaceutical composition, may be administered to mammals, including humans, either intravenously, subcutaneously, intramuscularly, intranasally, intracerebrospinally or orally. The peptides should be at least about 93% pure and preferably should have a purity of at least about 98%. This purity means that the intended peptide constitutes the stated weight % of all like peptides and peptide fragments present. Their administration may be employed by a physician to lower blood pressure or to stimulate endogenous gluco-corticoid production. The required dosage will vary with the particular condition being treated, with the severity of the condition and with the duration of desired treatment.

Such peptides are often administered in the form of pharmaceutically acceptable nontoxic salts, such as acid addition salts or metal complexes, e.g., with zinc, iron, calcium, barium, magnesium, aluminium or the like (which are considered as addition salts for purposes of this application). Illustrative of such acid addition salts are hydrochloride, hydrobromide, sulphate, phosphate, tannate, pamoate, oxalate, fumarate, gluconate, alginate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically-acceptable carrier. Usually, the dosage will be from about 1 to about 200 micrograms of the peptide per kilogram of the body weight of the host. In some instances, treatment of subjects with these peptides can be carried out in lieu of the administration of ACTH or corticosteroids, in such instances a dosage as low as about 10 ng/kg of body weight may be employed. As used herein all temperatures are °C and all ratios are by volume. Percentages of liquid materials are also by volume.

Although the invention has been described with regard to its preferred embodiments, which constitute the best mode presently known to the inventors, it should be understood that various changes and modifications as would be obvious to one having the ordinary skill in this art may be made without departing from the scope of the invention which is set forth in the claims appended hereto. For example, substitutions and modifications at other positions in the CRF peptide chain can be made in accordance with present or future developments without detracting from the potency of the analogs, and such peptides are considered as being within the scope of the invention.

## Claims

For the contracting states: BE, CH, DE, FR, GB, IT, LI, LU, NL, SE

1. A compound having the formula:
Z-$R_1$-Pro-Pro-Ile-Ser-$R_8$-Asp-Leu-$R_{11}$-$R_{12}$-$R_{13}$-Leu-Leu-Arg-$R_{17}$-$R_{18}$-$R_{19}$-Glu-$R_{21}$-$R_{22}$-Lys-$R_{24}$-$R_{25}$-$R_{26}$-$R_{27}$-$R_{28}$-Gln-Gln-Ala-$R_{32}$-$R_{33}$-Asn-Arg-$R_{36}$-Leu-Leu-Asp-$R_{40}$-$R_{41}$-$NH_2$
or a nontoxic addition salt thereof: wherein Z is an acyl group having 7 or less carbon atoms or hydrogen; $R_1$ is Ser-Gln-Glu or pGlu-Gly or Gln-Glu or Glu or D-Ser-Gln-Glu or D-pGlu-Gly or des $R_1$; $R_8$; $R_{12}$, $R_{19}$, $R_{24}$ and $R_{40}$ are selected from the group consisting of Leu, Ile, Ala, Gly, Val, Nle, Phe and Gln; $R_{11}$ is Thr or Ser; $R_{13}$ is His, Tyr or Glu; $R_{17}$ is Glu or Lys; $R_{18}$ is Val or Met; $R_{21}$ is Met, Met(O), Ile, Ala, Leu, Gly, Nle, Val, Phe or Gln; $R_{22}$ is Thr or Glu; $R_{25}$ is Asp or Glu; $R_{26}$ is Gln or Lys; $R_{27}$ is Leu, Ile, Ala, Gly, Val, Nle, Phe, Asp, Asn, Gln or Glu; $R_{28}$ is Ala or Lys; $R_{32}$ is His, Tyr or Ala; $R_{33}$ is Ser, Asn, Thr or Ala; $R_{36}$ is Lys or Leu; $R_{41}$ is Ala, Ile, Gly, Val, Leu, Nle, Phe, Gln or des $R_{41}$, provided however that when $R_{13}$ is His, then $R_{17}$ is

Glu, $R_{18}$ is Val, $R_{22}$ is Thr, $R_{26}$ is Gln, $R_{28}$ is Ala, and $R_{36}$ is Lys; and provied that when $R_{13}$ is Glu, $R_{17}$ is Lys, $R_{18}$ is Met, $R_{22}$ is Glu, $R_{26}$ is Lys, $R_{28}$ is Lys, $R_{32}$ is Ala and $R_{36}$ is Leu, then either $R_1$ is not pGlu-Gly or $R_8$ is not Ile or $R_{11}$ is Thr or $R_{12}$ is not Leu or $R_9$ is not Ile or $R_{21}$ is not Ile or $R_{24}$ is not Gln or $R_7$ is not Glu or $R_{33}$ is not Asn or $R_{40}$ ist not Thr or $R_{41}$ is not Ile and provided further that Z may optionally include 1 to 10 amino acids.

2. The compound of Claim 1 wherein $R_{13}$ is His.

3. The compound of Claim 1 wherein $R_{13}$ is Tyr.

4. The compound of Claim 2 or 3 wherein $R_{25}$ is Glu.

5. The compound of Claim 2 or 3 wherein $R_{25}$ is Asp.

6. The compound of Claim 1 wherein $R_{11}$ is Thr, $R_{13}$ is His, $R_{21}$ is Met, and $R_{36}$ is Lys.

7. The compound of Claim 1 having the formula:
H-Ser-Gln-Glu-Pro-Pro-Ile-Ser-Leu-Pro-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-NH$_2$
and being at least about 93% pure.

8. The compound of Claim 1 having the formula:

H-Pro-Pro-Ile-Ser-Ile-Asp-Leu-Ser-Leu-Glu-Leu-Leu-Arg-Lys-Met-Ile-Glu-Ile-Glu-Lys-Gln-Glu-Lys-Glu-Lys-Gln-Gln-Ala-Ala-Asn-Asn-Arg-Leu-Leu-Leu-Asp-Thr-Ile-Ile-NH$_2$.

9. A compound as claimed in Claim 1 and substantially as hereinbefore specifically identified in respect of Examples I through XVIII.

10. A pharmaceutical composition comprising a compound as claimed in any one of Claims 1 to 9 together with a carrier or diluent rendering the composition administrable orally, intravenously, subcutaneously, intranasally, intracerebrospinally or intramuscularly.

11. A peptide intermediate having the formula:

X$^1$-Ser(X$^2$)-Gln(X$^4$)-Glu(X$^5$)-Pro-Pro-Ile-Ser(X$^2$)-Leu-Asp(X$^5$)-Leu-Thr(X$^2$)-Phe-His(X$^8$)-Leu-Leu-Arg(X$^3$)-Glu(X$^5$)-Val-Leu-Glu(X$^5$)-Met(O)-Thr(X$^2$)-Lys(X$^6$)-Ala-Asp(X$^5$)-Gln(X$^4$)-Leu-Ala-Gln(X$^4$)-Gln(X$^4$)-Ala-His(X$^8$)-Ser(X$^2$)-Asn(X$^4$)-Arg(X$^3$)-Lys(X$^6$)-Leu-Leu-Asp(X$^5$)-Ile-Ala(X$^7$) wherein:

X$^1$ is hydrogen or an α-amino protecting group;
X$^2$ is hydrogen or a protecting group for the alcoholic hydroxyl group of a Ser or Thr residue;
X$^3$ is hydrogen or a protecting group for the guanidino group of Arg;
X$^4$ is hydrogen or a protecting group for the amido group of Gln or Asn;
X$^5$ is hydrogen or a protecting group for the carboxyl group of Asp or Glu;
X$^6$ is hydrogen or a protecting group for the side chain amino group of Lys;
X$^7$ is selected from the class consisting of OH, OCH$_3$, esters, amides, hydrazides, –O–CH$_2$–polystyrene resin support and –O–CH$_2$-benzyl-polystyrene resin support; and
X$^8$ is hydrogen or a protecting group for the imidazole nitrogen of His; provided that not all of X$^1$ to X$^8$ are hydrogen or OH.

**Claims**

For the contracting state: AT

1. A process for the manufacture of a peptide compound comprising:

(a) forming a peptide intermediate having the formula:

X$^1$-R$_1$-Pro-Pro-Ile-Ser(X$^2$)-R$_8$-Asp(X$^5$)-Leu-R$_{11}$(X$^2$)-R$_{12}$-R$_{13}$-Leu-Leu-Arg(X$^3$)-R$_{17}$-R$_{18}$-R$_{19}$-Glu(X$^5$)-R$_{21}$-R$_{22}$-Lys(X$^6$)-R$_{24}$-R$_{25}$-R$_{26}$-R$_{27}$-R$_{28}$-Gln(X$^4$)-Gln(X$^4$)-Ala-R$_{32}$-R$_{33}$-Asn(X$^4$)-Arg(X$^3$)-R$_{36}$-Leu-Leu-Asp(X$^5$)-R$_{40}$-R$_{41}$-X$^7$ wherein R$_1$ is Ser-Gln-Glu or pGlu-Gly or Gln-Glu or Glu or D-Ser-Gln-Glu or D-pGlu-Gly or des R$_1$; R$_8$, R$_{12}$, R$_{19}$, R$_{24}$ and R$_{40}$ are selected from the group consisting of Leu, Ile, Ala, Gly, Val, Nle, Phe and Gln; R$_{11}$ is Thr or Ser; R$_{13}$ is His, Tyr or Glu; R$_{17}$ is Glu or Lys; R$_{18}$ is Val or Met; R$_{21}$ is Met, Met(O), Ile, Ala, Leu, Gly, Nle, Val, Phe or Gln; R$_{22}$ is Thr or Glu; R$_{25}$ is Asp or Glu; R$_{26}$ is Gln or Lys; R$_{27}$ is Leu, Ile, Ala, Gly, Val, Nle, Phe, Asp, Asn, Gln, or Glu; R$_{28}$ is Ala or Lys; R$_{32}$ is His, Tyr or Ala; R$_{33}$ is Ser, Asn, Thr or Ala; R$_{36}$ is Lys or Leu; R$_{41}$ is Ala, Ile, Gly, Val, Leu, Nle, Phe, Gln or des R$_{41}$, provided however that when R$_{13}$ is His, then R$_{17}$ is Glu, R$_{18}$ is Val, R$_{22}$ is Thr, R$_{26}$ is Gln, R$_{28}$ is Ala, and R$_{36}$ is Lys; and provided that when R$_{13}$ is Glu, R$_{17}$ is Lys, R$_{18}$ is Met, R$_{22}$ is Glu, R$_{26}$ is Lys, R$_{28}$ is Lys, R$_{32}$ is Ala and R$_{36}$ is Leu, then either R$_1$ is not pGlu-Gly or R$_8$ is not Ile or R$_{11}$ is Thr or R$_{12}$ is not Leu or R$_{19}$ is not Ile or R$_{21}$ is not Ile or R$_{24}$ is not Gln or R$_{27}$ is not Glu or R$_{33}$ is not Asn or R$_{40}$ is not Thr or R$_{41}$ is not Ile, and wherein:

X$^1$ is hydrogen or an α-amino protecting group;
X$^2$ is hydrogen or a protecting group for the alcoholic hydroxyl group of a Ser or Thr residue;
X$^3$ is hydrogen or a protecting group for the guanidino group of Arg;
X$^4$ is hydrogen or a protecting group for the amido group of Gln or Asn;
X$^5$ is hydrogen or a protecting group for the carboxyl group of Asp or Glu;
X$^6$ is hydrogen or a protecting group for the side chain amino group of Lys;
X$^7$ is selected from the class consisting of OH, OCH$_3$, esters, amides, hydrazides,–O–CH$_2$–polystyrene resin support and –O–CH$_2$–benzyl-polystyrene resin support; provided that not all of X$^1$ to X$^7$ are hydrogen or OH,

(b) removing protecting groups and optional covalently bound resin support from said polypeptide intermediate to provide a polypeptide and, if desired converting such resulting polypeptide into a nontoxic salt thereof.

2. A method in accordance with Claim 1 wherein R$_{13}$ is His.

3. A method in accordance with Claim 1 wherein $R_{13}$ is Tyr.

4. A method in accordance with any of Claims 1, 2 and 3 wherein $R_{25}$ is Glu.

5. A method in accordance with any of Claims 1, 2 and 3 wherein $R_{25}$ is Asp.

6. A method in accordance with any of Claims 1 to 5 wherein $R_{11}$ is Tyr, $R_{21}$ is Met and $R_{36}$ is Lys.

7. A method in accordance with any of Claims 1 to 6 wherein $R_{12}$ is Phe.

8. A method in accordance with any of Claims 1 to 6 wherein $R_{12}$ is Leu.

9. A method in accordance with Claim 1 wherein said intermediate has the formula:

$X^1$-Ser($X^2$)-Gln($X^4$)-Glue($X^5$)-Pro-Pro-Ile-Ser($X^2$)-Leu-Asp($X^5$)-Leu-Thr($X^2$)-Phe-His($X^8$)-Leu-Leu-Arg($X^3$)-Glu($X^5$)-Val-Leu-Glu($X^5$)-Met(O)-Thr($X^2$)-Lys($X^6$)-Ala-Asp($X^5$)-Gln($X^4$)-Leu-Ala-Gln($X^4$)-Ala-His($X^8$)-Ser($X^2$)-Asn($X^4$)-Arg($X^3$)-Lys($X^6$)-Leu-Leu-Asp($X^5$)-Ile-Ala($X^7$)
wherein:

$X^8$ is hydrogen or a protecting group for the imidazole nitrogen of Hs.

10. A method in accordance with any of Claims 1 to 9 wherein $X^7$ is either –O–CH$_2$–polystyrene resin support or –O–CH$_2$–benzyl-polystrene resin support.

## Patentansprüche

für die Vertragsstaaten:
BE, FR, DE, IT, LI, LU, NL, SE, CH, GB,

1. Verbindung mit der allgemeinen Formel:
Z-$R_1$-Pro-Pro-Ile-Ser-$R_8$-Asp-Leu-$R_{11}$-$R_{12}$-$R_{13}$-Leu-Leu-Arg-$R_{17}$-$R_{18}$-$R_{19}$-Glu-$R_{21}$-$R_{22}$-Lys-$R_{24}$-$R_{25}$-$R_{26}$-$R_{27}$-$R_{28}$-Gln-Gln-Ala-$R_{32}$-$R_{33}$-Asn-Arg-$R_{36}$-Leu-Leu-Asp-$R_{40}$-$R_{41}$-NH$_2$
oder ein nichttoxisches Additionssalz, wobei Z eine Acylgruppe mit 7 oder weniger Kohlenstoffatomen oder Wasserstoff ist; $R_1$ Ser-Gln-Glu oder pGlu-Gly oder Gln-Glu oder Glu oder D-Ser-Gln-Glu oder D-pGlu-Gly oder des $R_1$, $R_8$, $R_{12}$, $R_{19}$, $R_{24}$ und $R_{40}$ ausgewählt sind aus der Gruppe bestehend aus Leu, Ile, Ala, Gly, Val, Nle, Phe und Gln; $R_{11}$ Thr oder Ser ist; $R_{13}$ Hs, Tyr oder Glu ist; $R_{17}$ Glu oder Lys ist; $R_{18}$ Val oder Met ist; $R_{21}$ Met, Met(O), Ile, Ala, Leu, Gly, Nle, Val, Phe oder Gln ist; $R_{22}$ Thr oder Glu ist; $R_{25}$ Asp oder Glu ist; $R_{26}$ Gln oder Lys ist, $R_{27}$ Leu, Ile, Ala, Gly, Val, Nle, Phe, Asp, Asn, Gln oder Glu ist, $R_{28}$ Ala oder Lys ist; $R_{32}$ His, Tyr oder Ala ist, $R_{33}$ Ser, Asn, Thr oder Ala ist; $R_{36}$ Lys oder Leu ist; $R_{41}$ Ala, Ile, Gly, Val, Leu, Nle, Phe, Gln oder des $R_{41}$ ist; vorausgesetzt, dass wenn $R_{13}$ His ist, $R_{17}$ Glu ist, $R_{18}$ Val ist, $R_{22}$ Thr ist, $R_{26}$ Gln ist, $R_{28}$ Ala ist und $R_{36}$ Lys ist; und vorausgesetzt, dass wenn $R_{13}$ Glu ist, $R_{17}$ Lys ist, $R_{18}$ Met ist, $R_{22}$ Glu ist, $R_{26}$ Lys ist, $R_{28}$ Lys ist, $R_{32}$ Ala ist und $R_{36}$ Leu ist, dass dann entweder $R_1$ nicht p-Glu-Gly ist oder $R_8$ nicht Ile oder $R_{11}$ Thr ist oder $R_{12}$ nicht Leu ist oder $R_{19}$ nicht Ile ist oder $R_{21}$ nicht Ile oder $R_{24}$ nicht Gln oder $R_{27}$ nicht Glu ist

oder $R_{33}$ nicht Asn ist oder $R_{40}$ nicht Thr ist oder $R_{41}$ nicht Ile ist und weiter vorausgesetzt, dass Z gegebenenfalls 1 bis 10 Aminosäuren einschliessen kann.

2. Verbindung nach Anspruch 1, dadurch gekennzeichnet, dass $R_{13}$ His ist.

3. Verbindung nach Anspruch 1, dadurch gekennzeichnet, dass $R_{13}$ Tyr ist.

4. Verbindung nach Anspruch 2 oder 3, dadurch gekennzeichnet, dass $R_{25}$ Glu ist.

5. Verbindung nach Anspruch 2 oder 3, dadurch gekennzeichnet, dass $R_{25}$ Asp ist.

6. Verbindung nach Anspruch 1, dadurch gekennzeichnet, dass $R_{11}$ Thr ist, $R_{13}$ His ist, $R_{21}$ Met ist und $R_{36}$ Lys ist.

7. Verbindung nach Anspruch 1 mit der allgemeinen Formel
H-Ser-Gln-Glu-Pro-Pro-Ile-Ser-Leu-Asp-Leu-Thr-Phe-His-Leu-Leu-Arg-Glu-Val-Leu-Glu-Met-Thr-Lys-Ala-Asp-Gln-Leu-Ala-Gln-Gln-Ala-His-Ser-Asn-Arg-Lys-Leu-Leu-Asp-Ile-Ala-NH$_2$
und dass sie zumindestens 93% rein ist.

8. Verbindung nach Anspruch 1 mit der allgemeinen Formel
H-Pro-Pro-Ile-Ser-Ile-Asp-Leu-Ser-Leu-Glu-Leu-Leu-Arg-Lys-Met-Ile-Glu-Ile-Glu-Lys-Gln-Glu-Lys-Glu-Lys-Gln-Gln-Ala-Ala-Asn-Asn-Arg-Leu-Leu-Leu-Asp-Thr-Ile-NH$_2$.

9. Verbindung wie beansprucht in Anspruch 1 und wie im wesentlichen vorher identifiziert insbesondere in den Beispielen I bis XVIII.

10. Zusammensetzung bestehend aus einer Verbindung nach einem der Ansprüche 1 bis 9 in Kombination mit einem Träger oder einem Verdünnungsmittel, das die Zusammensetzung oral, intravenös, subcutan, intranasal, intracerebrospinal oder intramuskulär anwendbar macht.

11. Peptid-Zwischenprodukt mit der allgemeinen Formel $X^1$-Ser ($X^2$)-Gln ($X^4$)-Glu ($X^5$)-Pro-Pro-Ile-Ser($X_2$)-Leu-Asp ($X^5$)-Leu-Thr- ($X^2$)-Phe-His ($X^8$)-Leu-Leu-Arg($X^3$)-Glu ($X^5$)-Val-Leu-Glu ($X^5$)-Met(O)-Thr ($X^2$)-Lys ($X^6$)-Ala-Asp ($X^5$)-Gln ($X^4$)-Leu-Ala-Gln ($X^4$)-Gln ($X^4$)-Gln ($X^4$)-Ala-His ($X^8$)-Ser ($X^2$)-Asn ($X^4$)-Arg ($X^3$)-Lys ($X^6$)-Leu-Leu-Asp ($X^5$)-Ile-Ala-($X^7$)
worin bedeutet:

$X^1$ Hydrogen oder eine alpha-Amino-Schutzgruppe;
$X^2$ Wasserstoff oder eine Schutzgruppe für die alkoholische Hydroxylgruppe eines Ser- oder Thr-Restes;
$X^3$ Wasserstoff oder eine Schutzgruppe für die Guanidinogruppe von Arg;
$X^4$ Wasserstoff oder eine Schutzgruppe für die Amidogruppe von Gln oder Asn;
$X^5$ Wasserstoff oder eine Schutzgruppe für die Carboxylgruppe von Asp oder Glu;
$X^6$ Wasserstoff oder eine Schutzgruppe für die Seitenkettenaminogruppe von Lys;
$X^7$ ausgewählt ist aus der Klasse, bestehend aus OH, OCH$_3$, Estern, Amiden, Hydraziden, –O–CH$_2$-Polystyrolharzträger und –O–CH$_2$-Benzyl-Polystyrolharzträger; und
$X^8$ Wasserstoff oder eine Schutzgruppe für den Imidazolstickstoff von His; vorausgesetzt, dass nicht alle X¹ bis X⁸ Wasserstoff oder OH bedeuten.

**Patentansprüche**

für den Vertragsstaat: AT

1. Verfahren zur Herstellung einer Peptidverbindung durch

a) Bildung eines Peptidzwischenprodukts mit der Formel:
$X^1$-$R_1$-Pro-Pro-Ile-Ser($X^2$)-$R_8$-Asp($X^5$)-Leu-$R_{11}$
($X^2$)-$R_{12}$-$R_{13}$-Leu-Leu-Arg($X^3$)-$R_{17}$-$R_{18}$-$R_{19}$-Glu
($X^5$)-$R_{21}$-$R_{22}$-Lys($X^6$)-$R_{24}$-$R_{25}$-$R_{26}$-$R_{27}$-$R_{28}$
-Gln($X^4$)-Gln($X^4$)-Ala-$R_{32}$-$R_{33}$-Asn($X^4$)-Arg($X^3$)
-$R_{36}$-Leu-Leu-Asp($X^5$)-$R_{40}$-$R_{41}$-$X^7$,
worin $R_1$ Ser-Gln-Glu oder pGlu-Gly oder Gln-Glu oder Glu oder D-Ser-Gln-Glu oder D-pGlu-Gly oder des $R_1$ bedeutet; $R_8$, $R_{12}$, $R_{19}$, $R_{24}$ und $R_{40}$ ausgewählt sind aus der Gruppe bestehend aus Leu, Ile, Ala, Gly, Val, Nle, Phe und Gln; $R_{11}$ Thr oder Ser ist; $R_{13}$ His, Tyr oder Glu ist; $R_{17}$ Glu oder Lys ist; $R_{18}$ Val oder Met ist; $R_{21}$ Met, Met(O), Ile, Ala, Leu, Gly, Nle, Val, Phe oder Gln ist; $R_{22}$ Thr oder Glu ist; $R_{25}$ Asp oder Glu ist; $R_{26}$ Gln oder Lys ist; $R_{27}$ Leu, Ile, Ala, Gly, Val, Nle, Phe, Asp, Asn, Gln oder Glu ist; $R_{28}$ Ala oder Lys ist; $R_{32}$ His, Tyr oder Ala ist; $R_{33}$ Ser, Asn, Thr oder Ala ist; $R_{36}$ Lys oder Leu ist; $R_{41}$ Ala, Ile, Gly, Val, Leu, Nle, Phe, Gln oder des $R_{41}$ ist; vorausgesetzt jedoch, dass wenn $R_{13}$ His ist, dann $R_{17}$ Glu ist, $R_{18}$ Val ist, $R_{22}$ Thr ist, $R_{26}$ Gln ist, $R_{28}$ Ala ist und $R_{36}$ Lys ist; und vorausgesetzt, dass wenn $R_{13}$ Glu ist, $R_{17}$ Lys ist, $R_{18}$ Met ist, $R_{22}$ Glu ist, $R_{26}$ Lys ist, $R_{28}$ Lys ist, $R_{32}$ Ala ist und $R_{36}$ Leu ist, dass dann entweder $R_1$ nicht pGlu-Gly oder $R_8$ nicht Ile oder $R_{11}$ nicht Thr oder $R_{12}$ nicht Leu oder $R_{19}$ nicht Ile oder $R_{21}$ nicht Ile oder $R_{24}$ nicht Gln oder $R_{27}$ nicht Glu oder $R_{33}$ nicht Asn oder $R_{40}$ nicht Thr oder $R_{41}$ nicht Ile ist und worin

$X^1$ Wasserstoff oder eine alpha-Aminoschutzgruppe ist;
$X^2$ Wasserstoff oder eine Schutzgruppe für die alkoholische Hydroxylgruppe von einem Ser- oder Thr-Rest ist;
$X^3$ Wasserstoff oder eine Schutzgruppe für die Guanidinogruppe von Arg ist;
$X^4$ Wasserstoff oder eine Schutzgruppe für die Amidogruppe von Gln odere Asn ist;
$X^5$ Wasserstoff oder eine Schutzgruppe für die Carboxylgruppe von Asp oder Glu ist;
$X^6$ Wasserstoff oder eine Schutzgruppe für die Seitenkettenaminogruppe von Lys ist;
$X^7$ ausgewählt ist aus der Klasse bestehend aus OH, $OCH_3$, Estern, Amiden, Hydrziden, $O$–$CH_2$-Polystyrolharzträger und –$O$–$CH_2$-Benzyl-Polystyrolharzträger; vorausgesetzt, dass nicht alle $X^1$ bis $X^7$ Wasserstoff oder OH sind,

b) Entfernen der Schutzgruppen und gegebenenfalls des kovalent gebundenen Harzträgers von dem Polypeptidzwischenprodukt um ein Polypeptid zu schaffen und falls gewünscht, das entstehende Polypeptid in ein nichttoxisches Salz umzuwandeln.

2. Verfahren nach Anspruch 1, dadurch gekennzeichnet, das $R_{13}$ His ist.

3. Verfahren nach Anspruch 1, dadurch gekennzeichnet, dass $R_{13}$ Tyr ist.

4. Verfahren nach Anspruch 1, 2 oder 3, dadurch gekennzeichnet, dass $R_{25}$ Glu ist.

5. Verfahren nach einem der Ansprüche 1, 2 oder 3, dadurch gekennzeichnet, dass $R_{25}$ Asp ist.

6. Verfahren nach einem der Ansprüche 1 bis 5, dadurch gekennzeichnet, dass $R_{11}$ Tyr ist, $R_{21}$ Met ist und $R_{36}$ Lys ist.

7. Verfahren nach einem der Ansprüche 1 bis 6, dadurch gekennzeichnet, dass $R_{12}$ Phe ist.

8. Verfahren nach einem der Ansprüche 1 bis 6, dadurch gekennzeichnet, dass $R_{12}$ Leu ist.

9. Verfahren nach Anspruch 1, dadurch gekennzeichnet, dass das Zwischenprodukt die folgende Formel hat:

$X^1$-Ser($X^2$)-Gln($X^4$)-Glue($X^5$)-Pro-Pro-Ile-Ser($X^2$)-Leu-Asp($X^5$)-Leu-Thr($X^2$)-Phe-His($X^8$)-Leu-Leu-Arg($X^3$)-Glu($X^5$)-Val-Leu-Glu($X^5$)-Met(O)-Thr($X^2$)-Lys($X^6$)-Ala-Asp($X^5$)-Gln($X^4$)-Leu-Ala-Gln($X^4$)-Gln($X^4$)-Ala-His($X^8$)-Ser($X^2$)-Asn($X^4$)-Arg($X^3$)-Lys($X^6$)-Leu-Leu-Asp($X^5$)-Ile-Ala($X^7$)
worin $X^8$ Wasserstoff oder eine Schutzgruppe für den Imidazolstickstoff von His bedeutet.

10. Verfahren nach einem der Ansprüche 1 bis 9, dadurch gekennzeichnet, dass $X^7$ entweder –$O$–$CH_2$-Polystyrolharzträger oder –$O$–$CH_2$-Benzyl-Polystyrolharzträger ist.

**Patentansprüche**

für den Vertragsstaat: AT

1. Verfahren zur Herstellung einer Peptidverbindung durch

a) Bildung eines Peptidzwischenprodukts mit der Formel:
$X^1$-$R_1$-Pro-Pro-Ile-Ser($X^2$-$R_8$-Asp($X^5$)-Leu-R
($X^2$)-$R_{12}$-$R_{13}$-Leu-Leu-Arg($X^3$)-$R_{17}$-$R_{18}$-$R_{19}$-Glu
($X^5$)-$R_{21}$-$R_{-2}$-Lys($X^5$)-$R_{24}$-$R_{25}$-$R_{26}$-$R_{27}$-$R_{28}$
-Gln($X^4$)-Gln($X^4$)-Ala-$R_{32}$-Asn($X^4$)-Ala-$R_{32}$-Asn
($X^4$)-Arg($X^3$)-$R_{36}$-Leu-Leu-Asp($X^5$)-$R_{40}$-$R_{-R41}$-$X^7$,
worin $R_1$ Ser-Gln-Glu oder pGlu-Gly oder Gln-Glu oder Glu oder D-Ser-Gln-Glu oder D-pGlu-Gly oder des $R_1$ bedeutet; $R_8$, $R_{12}$, $R_{19}$, $R_{24}$ und $R_{40}$ ausgewählt sind aus der Gruppe bestehend aus Leu, Ile, Ala, Gly, Val, Nle, Phe und Gln; $R_{11}$ Thr oder Ser ist; $R_{13}$ His, Tyr oder Glu ist; $R_{17}$ Glu oder Lys ist; $R_{18}$ Val oder Met ist; $R_{21}$ Met, Met(O), Ile, Ala, Leu, Gly, Nle, Val, Phe oder Gln ist; $R_{22}$ Thr oder Glu ist; $R_{25}$ Asp oder Glu ist; $R_{26}$ Gln oder Lys ist; $R_{27}$ Leu, Ile, Ala, Gly, Val, Nle, Phe, Asp, Asn, Gln oder Glu ist; $R_{28}$ Ala oder Lys ist; $R_{23}$ His, Tyr oder Ala ist; $R_{33}$ Ser, Asn, Thr oder Ala ist; $R_{36}$ Lys oder Leu ist; $R_{41}$ Ala, Ile, Gly, Val, Leu, Nle, Phe, Gln oder des $R_{41}$ ist; vorausgesetzt jedoch, dass wenn $R_{13}$ His ist, dann $R_{17}$ Glu ist, $R_{18}$ Val ist, $R_{22}$ Thr ist,

$R_{26}$ Gln ist, $R_{28}$ Ala ist und $R_{36}$ Lys ist; und vorausgesetzt, dass wenn $R_{13}$ Glu ist, $R_{17}$ Lys ist, $R_{18}$ Met ist, ist, $R_{22}$ Glu ist, $R_{26}$ Lys ist, $R_{28}$ Lys ist, $R_{32}$ Ala ist und $R_{36}$ Leu ist, dass dann entweder $R_1$ nicht pGlu-Gly oder $R_8$ nicht Ile oder $R_{11}$ nicht Thr oder $R_{12}$ nicht Leu oder $R_{19}$ nicht Ile oder $R_{21}$ nicht Ile oder $R_{24}$ nicht Gln oder $R_{27}$ nicht Glu oder $R_{33}$ nicht Asn oder $R_{40}$ nicht Thr oder $R_{41}$ nicht Ile ist und worin

$X^1$ Wasserstoff oder eine alpha-Aminoschutzgruppe ist;
$X^2$ Wasserstoff oder eine Schutzgruppe für die alkoholische Hydroxylgruppe von einem Ser- oder Thr-Rest ist;
$X^3$ Wasserstoff oder eine Schutzgruppe für die Guanidinogruppe von Arg ist;
$X^4$ Wasserstoff oder eine Schutzgruppe für die Amidogruppe von Gln oder Asn ist;
$X^5$ Wasserstoff oder eine Schutzgruppe für die Carboxylgruppe von Asp oder Glu ist;
$X^6$ Wasserstoff oder eine Schutzgruppe für die Seitenkettenaminogruppe von Lys ist;
$X^7$ ausgewählt ist aus der Klasse bestehend aus OH, $OCH_3$, Estern, Amiden, Hydraziden, $-O-CH_2$-Polystyrolharzträger und $-O-CH_2$-Benzyl-Polystyrolharzträger; vorausgesetzt, dass nicht alle $X^1$ bis $X^7$ Wasserstoff oder OH sind,

b) Entfernen der Schutzgruppen und gegebenenfalls des kovalent gebundenen Harzträgers von dem Polypeptidzwischenprodukt um ein Polypeptid zu schaffen und falls gewünscht, das entstehende Polypeptid in ein nichttoxisches Salz umzuwandeln.

2. Verfahren nach Anspruch 1, dadurch gekennzeichnet, dass $R_{13}$ His ist.
3. Verfahren nach Anspruch 1, dadurch gekennzeichnet, dass $R_{13}$ Tyr ist.
4. Verfahren nach Anspruch 1, 2 oder 3, dadurch gekennzeichnet, dass $R_{25}$ Glu ist.
5. Verfahren nach einem der Ansprüche 1, 2 oder 3, dadurch gekennzeichnet, dass $R_{25}$ Asp ist.
6. Verfahren nach einem der Ansprüche 1 bis 5, dadurch gekennzeichnet, dass $R_{11}$ Tyr ist, $R_{21}$ Met ist und $R_{36}$ Lys ist.
7. Verfahren nach einem der Ansprüche 1 bis 6, dadurch gekennzeichnet, dass $R_{12}$ Phe ist.
8. Verfahren nach einem der Ansprüche 1 bis 6, dadurch gekennzeichnet, dass $R_{12}$ Leu ist.
9. Verfahren nach Anspruch 1, dadurch gekennzeichnet, dass das Zwischenprodukt die folgende Formel hat:

$X^1$-Ser($X^2$)-Gln($X^4$)-Glue($X^5$)-Pro-Pro-Ile-Ser ($X^2$)-Leu-Asp($X^5$)-Leu-Thr($X^2$)-Phe-His($X^8$)-Leu-Leu-Arg($X^3$)-Glu($X^5$)-Val-Leu-Glu($X^5$)-Met(O)-Thr ($X^2$)-Lys($X^6$)-Ala-Asp($X^5$)-Gln($X^4$)-Leu-Ala-Gln ($X^4$)-Gln($X^4$)-Ala-His($X^8$)-Ser($X^2$)-Asn($X^4$)-Arg($X^3$)-Lys($X^6$)-Leu-Leu-Asp($X^5$)-Ile-Ala($X^7$)
worin $X^8$ Wasserstoff oder eine Schutzgruppe für den Imidazolstickstoff von His bedeutet.
10. Verfahren nach einem der Ansprüche 1 bis 9, dadurch gekennzeichnet, dass $X^7$ entweder $-O-CH_2$-Polystyrolharzträger oder $-O-CH_2$-Benzyl-Polystyrolharzträger ist.

**Revendications**

pour les Etats contractants: BE, FR, DE, IT, LU, LI, NL, SE, CH, GB

1. Un composé ayant pour formule:
Z-$R_1$-Pro-Pro-Ile-Sér-$R_8$-Asp-Leu-$R_{11}$-$R_{12}$-$R_{13}$-Leu-Leu-Arg-$R_{17}$-$R_{18}$-$R_{19}$-Glu-$R_{21}$-$R_{22}$-Lys-$R_{24}$-$R_{25}$-$R_{26}$-$R_{27}$-$R_{28}$-Gln-Gln-Ala-$R_{32}$-$R_{33}$-Asn-Arg-$R_{36}$-Leu-Leu-Asp-$R_{40}$-$R_{41}$-$NH_2$

ou un sel d'addition non toxique correspondant; où Z est un groupe acyle ayant 7 atomes de carbone ou moins ou un hydrogène; $R_1$ est Sér-Gln-Glu ou pGlu-Gly ou Gln-Glu ou Glu ou D-Sér-Gln-Glu ou D-pGlu-Gly ou dés $R_1$; $R_8$, $R_{12}$, $R_{19}$, $R_{24}$ et $R_{40}$ sont choisis parmi le groupe constitué par Leu, Ile, Ala, Gly, Val, Nle, Phé et Gln; $R_{11}$ est Thr ou Sér; $R_{13}$ est His, Tyr ou Glu; $R_{17}$ est Glu ou Lys; $R_{18}$ est Val ou Mét; $R_{21}$ est Mét, Mét(O), Ile, Ala, Leu, Gly, Nle, Val, Phé ou Gln; $R_{22}$ est Thr ou Glu; $R_{25}$ est Asp ou Glu; $R_{26}$ est Gln ou Lys; $R_{27}$ est Leu, Ile, Ala, Gly, Val, Nle, Phé, Asp, Asn, Gln ou Glu; $R_{28}$ est Ala ou Lys; $R_{32}$ est His, Tyr ou Ala; $R_{33}$ est Sér, Asn, Thr ou Ala; $R_{36}$ est Lys ou Leu; $R_{41}$ est Ala, Ile, Gly, Val, Leu, Nle, Phé, Gln ou dés $R_{41}$, sous réserve cependant que lorsque $R_{13}$ est His, alors $R_{17}$ est Glu, $R_{18}$ est Val, $R_{22}$ est Thr, $R_{26}$ est Gln, $R_{28}$ est Ala et $R_{36}$ est Lys; et sous réserve que lorsque $R_{13}$ est Glu, $R_{17}$ est Lys, $R_{18}$ est Mét, $R_{22}$ est Glu, $R_{26}$ est Lys, $R_{28}$ est Lys, $R_{32}$ est Ala et $R_{36}$ est Leu, alors soit $R_1$ n'est pas pGlu-Gly, soit $R_8$ n'est pas Ile, soit $R_{11}$ est Thr, soit $R_{12}$ n'est pas Leu, soit $R_{19}$ n'est pas Ile, soit $R_{21}$ n'est pas Ile, soit $R_{24}$ n'est pas Gln, soit $R_{27}$ n'est pas Glu, soit $R_{33}$ n'est pas Asn, soit $R_{40}$ n'est pas Thr, soit $R_{41}$ n'est pas Ile et sous réserve de plus que Z peut éventuellement comprendre 1 à 10 amino-acides.
2. Le composé de la revendication 1, dans lequel $R_{13}$ est His.
3. Le composé de la revendication 1, dans lequel $R_{13}$ est Tyr.
4. Le composé de la revendication 2 ou 3, dans lequel $R_{25}$ est Glu.
5. Le composé de la revendication 2 ou 3, dans lequel $R_{25}$ est Asp.
6. Le composé de la revendication 1, dans lequel $R_{11}$ est Thr, $R_{13}$ est His, $R_{21}$ est Mét et $R_{36}$ est Lys.
7. Le composé de la revendication 1 ayant pour formule:
H-Sér-Gln-Glu-Pro-Pro-Ile-Sér-Leu-Asp-Leu-Thr-Phé-His-Leu-Leu-Arg-Glu-Val-Leu-Glu-Mét-Thr-Lys-Ala-Asp-Gln-Leu-Ala-Gln-Gln-Ala-His-Sér-Asn-Arg-Lys-Leu-Leu-Asp-Ile-Ala-$NH_2$
et étant pur au moins à environ 93%.
8. Le composé de la revendication 1, ayant pour formule:
H-Pro-Pro-Ile-Sér-Ile-Asp-Leu-Sér-Leu-Glu-Leu-Leu-Arg-Lys-Mét-Ile-Glu-Ile-Glu-Lys-Gln-Glu-Lys-Glu-Lys-Gln-Gln-Ala-Ala-Asn-Asn-Arg-Leu-Leu-Leu-Asp-Thr-Ile-$NH_2$.

9. Un composé comme revendiqué dans la revendication 1, et essentiellement comme précédemment identifié spécifiquement relativement aux exemples I à XVIII.

10. Une composition pharmaceutique comprenant un composé comme revendiqué dans l'une quelconque des revendications 1 à 9, avec un support ou diluant permettant que la composition puisse être administrée par voie orale, intraveineuse, souscutanée, intranasale, intracéphalorachidienne ou intramusculaire.

11. Un intermédiaire de peptide ayant pour formule:

$X^1$-Sér($X^2$)-Gln($X^4$)-Glu($X^5$)-Pro-Pro-Ile-Sér ($X^2$)-Leu-Asp($X^5$)-Leu-Thr($X^2$)-Phé-His($X^8$)-Leu-Leu-Arg($X^3$)-Glu($X^5$)-Val-Leu-Glu($X^5$)-Mét(O)-Thr ($X^2$)-Lys($X^6$)-Ala-Asp($X^5$)-Gln($X^4$)-Leu-Ala-Gln ($X^4$)-Gln($X^4$)-Ala-His($X^8$)-Sér($X^2$)-Asn($X^4$)-Arg ($X^3$)-Lys($X^6$)-Leu-Leu-Asp($X^5$)-Ile-Ala-($X^7$) dans laquelle

$X^1$ est un hydrogène ou un groupe $\alpha$-amino-protecteur;
$X^2$ est un hydrogène ou un groupe protecteur du groupe hydroxy alcoolique d'un reste Sér ou Thr;
$X^3$ est un hydrogène ou un groupe protecteur du groupe guanidino de Arg;
$X^4$ est un hydrogène ou un groupe protecteur du groupe amido de Gln ou Asn;
$X^5$ est un hydrogène ou un groupe protecteur du groupe carboxy de Asp ou Glu;
$X^6$ est un hydrogène ou un groupe protecteur du groupe amino de la chaîne latérale de Lys;
$X^7$ est choisi parmi la catégorie constituée par OH, $OCH_3$, les esters, les amides, les hydrazides, -O-$CH_2$-résine support de polystyrène et -O-$CH_2$-benzyl-résine support de polystyrène; et
$X^8$ est un hydrogène ou un groupe protecteur de l'azote de l'imidazole de His; sous réserve que tous les symboles $X^1$ à $X^8$ ne soient pas un hydrogène ou OH.

## Revendications

pour l'Etat contractant: AT

1. Un procédé pour la fabrication d'un composé peptidique comprenant: (a) la formation d'un peptide intermédiaire ayant pour formule:

$X^1$-$R_1$-Pro-Pro-Ile-Sér($X^2$)-$R_8$-Asp($X^5$)-Leu-$R_{11}$($X^2$) $R_{-12}$-$R_{13}$-Leu-Leu-Arg($X^3$)-$R_{17}$-$R_{18}$-$R_{19}$-Glu ($X^5$)-$R_{21}$-$R_{22}$-Lys($X^6$)$R_{24}$-$R_{25}$-$R_{26}$-$R_{27}$-$R_{28}$-Gln ($X^4$)-Gln($X^4$)-Ala-$R_{32}$-$R_{33}$-Asn($X^4$)-Arg($X^3$)-$R_{36}$-Leu-Leu-Asp($X^5$)-$R_{40}$-$R_{41}$-$X^7$

dans laquelle $R_1$ est Sér-Gln-Glu ou pGlu-Gly ou Gln-Glu ou Glu ou D-Sér-Gln-Glu ou D-pGlu-Gly ou dés $R_1$; $R_8$, $R_{12}$, $R_{19}$, $R_{24}$ et $R_{40}$ sont choisis parmi le groupe constitué par Leu, Ile, Ala, Gly, Val, Nle, Phé et Gln; $R_{11}$ est Thr ou Sér; $R_{13}$ est His, Tyr ou Glu; $R_{17}$ est Glu ou Lys; $R_{18}$ est Val ou Mét; $R_{21}$ est Mét(O), Ile, Ala, Leu, Gly, Nle, Val, Phé ou Gln; $R_{22}$ est Thr ou Glu; $R_{25}$ est Asp ou Glu; $R_{26}$ Gln ou Lys;

$R_{27}$ est Leu, Ile, Ala, Gly, Val, Nle, Phé, Asp, Asn, Gln ou Glu; $R_{28}$ est Ala ou Lys; $R_{32}$ est His, Tyr ou Ala; $R_{33}$ est Sér, Asn, Thr ou Ala; $R_{36}$ est Lys ou Leu; $R_{41}$ est Ala, Ile, Gly, Val, Leu, Nle, Phé, Gln ou dés $R_{41}$, sous réserve cependant que lorsque $R_{13}$ est His, alors $R_{17}$ est Glu, $R_{18}$ est Val, $R_{22}$ est Thr, $R_{26}$ est Gln, $R_{28}$ est Ala et $R_{36}$ est Lys; et sous réserve que lorsque $R_{13}$ est Glu, $R_{17}$ est Lys, $R_{18}$ est Mét, $R_{22}$ est Glu, $R_{26}$ est Lys, $R_{28}$ est Lys, $R_{32}$ est Ala et $R_{36}$ est Leu, alors soit $R_1$ n'est pas pGlu-Gly, soit $R_8$ n'est pas Ile, soit $R_{11}$ est Thr, soit $R_{12}$ n'est pas Leu, soit $R_{19}$ n'est pas Ile, soit $R_{21}$ n'est pas Ile, soit $R_{24}$ n'est pas Gln, soit $R_{27}$ n'est pas Glu, soit $R_{33}$ n'est pas Asn, soit $R_{40}$ n'est pas Thr, soit $R_{41}$ n'est pas Ile; et où

$X^1$ est un hydrogène ou un groupe $\alpha$-amino-protecteur;
$X^2$ est un hydrogène ou un groupe protecteur du groupe hydroxy alcoolique d'un reste Sér ou Thr;
$X^3$ est un hydrogène ou un groupe protecteur du groupe guanidino de Arg;
$X^4$ est un hydrogène ou un groupe protecteur du groupe amido du Gln ou Asn;
$X^5$ est un hydrogène ou un groupe protecteur du groupe carboxy d'Asp ou de Glu;
$X^6$ est un hydrogène ou un groupe protecteur du groupe amino de la chaîne latérale de Lys;
$X^7$ est choisi parmi la catégorie constituée par OH, $OCH_3$, les esters, les amides, les hydrazides, -O-$CH_2$-résine support de polystyrène et -O-$CH_2$-benzyl-résine support de polystyrène; sous réserve que tous les symboles $X^1$ à $X^7$ ne soient pas un hydrogène ou OH,

b) l'élimination des groupes protecteurs et de la résine support éventuelle liée par covalence dudit intermédiaire de polypeptide pour fournir un polypeptide et, si on le désire, la conversion du polypeptide obtenu en un de ses sels non toxiques.

2. Un procédé selon la revendication 1, dans lequel $R_{13}$ est His.
3. Un procédé selon la revendication 1, dans lequel $R_{13}$ est Tyr.
4. Un procédé selon l'une quelconque des revendications 1, 2 et 3, dans lequel $R_{25}$ est Glu.
5. Un procédé selon l'une quelconque des revendications 1, 2 et 3, dans lequel $R_{25}$ est Asp.
6. Un procédé selon l'une quelconque des revendications 1 à 5, dans lequel $R_{11}$ est Tyr, $R_{21}$ est Mét et $R_{36}$ est Lys.
7. Un procédé selon l'une quelconque des revendications 1 à 6, dans lequel $R_{12}$ est Phé.
8. Un procédé selon l'une quelconque des revendications 1 à 6, dans lequel $R_{12}$ est Leu.
9. Un procédé selon la revendication 1, dans lequel ledit intermédiaire a pour formule:

$X^1$-Sér($X^2$)-Gln($X^4$)-Glu($X^5$)-Pro-Pro-Ile-Sér ($X^2$)-Leu-Asp($X^5$)-Leu-Thr($X^2$)-Phé-His($X^8$)-Leu-Leu-Arg($X^3$)-Glu($X^5$)-Val-Leu-Glu($X^5$)-Mét(O)-Thr ($X^2$)-Lys($X^6$)-Ala-Asp($X^5$)-Gln($X^4$)-Leu-Ala-Gln ($X^4$)-Gln($X^4$)-Ala-His($X^8$)-Sér($X^2$)-Asn($X^4$)-Arg

(X³)-Lys(X⁶)-Leu-Leu-Asp(X⁵)-Ile-Ala(X⁷) où:

X⁸ est un hydrogène ou un groupe protecteur de l'azote de l'imidazole de His.

10. Un procédé selon l'une quelconque des revendications 1 à 9, dans lequel X⁷ est soit –O–CH₂-résine support de polystyrène, soit –O–CH₂-benzyl-résine support de polystyrène.